# EUROPEAN PATENT SPECIFICATION

(11) **EP 4 056 718 B1**
(45) Date of publication and mention of the grant of the patent: **18.09.2024**
(21) Application number: 22161710.3
(22) Date of filing: 11.03.2022
(51) Int. Cl.: C12Q 1/70

(54) **COMPOSITION FOR DETECTION OF SARS-COV-2 VIRUS GENE AND COVID-19 DIAGNOSISMETHOD USING REAL-TIME RT-PCR**
ZUSAMMENSETZUNG ZUM NACHWEIS DES SARS-COV-2-VIRUSGENS UND COVID-19-DIAGNOSEVERFAHREN UNTER VERWENDUNG VON ECHTZEIT-RT-PCR
COMPOSITION POUR LA DÉTECTION DU GÈNE DU VIRUS SARS-COV-2 ET PROCÉDÉ DE DIAGNOSTIC DU COVID-19 PAR RT-PCR EN TEMPS RÉEL

(30) Priority: 12.03.2021 KR 20210032898
(43) Date of publication of application: 14.09.2022
(73) Proprietor: Industry-Academic Cooperation Foundation, Chosun University, Gwangju 61452 (KR)
(72) Inventor: KIM, Dong Min, 61704 Gwangju (KR); LEE, You Mi, 61736 Gwangju (KR); KIM, Choon Mee, 61414 Gwangju (KR); HWANG, Seong Yeon, 61071 Gwangju (KR)
(74) Representative: Murgitroyd & Company

(56) References cited:
- CN-A- 111 270 021
- CN-A- 112 195 275
- YU SANGHEON ET AL: "Development of a Lateral Flow Strip Membrane Assay for Rapid and Sensitive Detection of the SARS-CoV-2", ANALYTICAL CHEMISTRY, vol. 92, no. 20, 23 September 2020 (2020-09-23), US, pages 14139 - 14144, XP055944090, ISSN: 0003-2700, DOI: 10.1021/acs.analchem.0c03202
- YU SANGHEON ET AL: "Supporting information Development of a Lateral Flow Strip Membrane Assay for Rapid and Sensitive Detection of the SARS-CoV-2", 23 September 2020 (2020-09-23), pages 1 - 6, XP055944106, Retrieved from the Internet <URL:https://pubs.acs.org/doi/suppl/10.1021/acs.analchem.0c03202/suppl_file/ac0c03202_si_001.pdf> [retrieved on 20220719]
- ANNAMALAI PAZHANIMUTHU ET AL: "A Simple Colorimetric Molecular Detection of Novel Coronavirus (COVID-19), an Essential Diagnostic Tool for Pandemic Screening", MEDRXIV, 14 April 2020 (2020-04-14), XP055892461, Retrieved from the Internet <URL:https://www.medrxiv.org/content/10.1101/2020.04.10.20060293v1.full.pdf> [retrieved on 20220216], DOI: 10.1101/2020.04.10.20060293
- KAILASA SURESH KUMAR ET AL: "An overview of molecular biology and nanotechnology based analytical methods for the detection of SARS-CoV-2: promising biotools for the rapid diagnosis of COVID-19", ANALYST, vol. 146, no. 5, 1 January 2021 (2021-01-01), UK, pages 1489 - 1513, XP055796101, ISSN: 0003-2654, Retrieved from the Internet <URL:https://pubs.rsc.org/en/content/articlepdf/2021/an/d0an01528h> DOI: 10.1039/D0AN01528H

## Description

### BACKGROUND OF THE INVENTION

### 1. Field of the invention

The present disclosure relates to a composition for detection of a SARS-CoV-2 virus gene, the composition including: the primer of SEQ ID NO: 1, the primer of SEQ ID NO: 2, and the probe of SEQ ID NO: 3; or the primer of SEQ ID NO: 4, the primer of SEQ ID NO: 5, and the probe of SEQ ID NO: 6, a kit comprising such a composition and a method for providing information for diagnosis of COVID-19, the method comprising the steps of:
(1) isolating a nucleic acid from a specimen from a patient suspected of COVID-19;
(2) performing PCR amplification with the diagnostic kit
   , with the isolated nucleic acid serving as a template; and
(3) identifying RdRp or ORF3a gene of SARS-CoV-2 in the amplicon obtained in step (2).

### 2. Description of the Prior Art

The SARS-CoV-2 virus was identified as the causative pathogen for pneumonia in Wuhan, China in 2019, with 78 million infected worldwide. The number of confirmed patients in Korea also increased rapidly, hitting 52,550 cases as of December 23, 2020.

Coronavirus disease 2019 (COVID-19) can be diagnosed and confirmed using real-time reverse transcription PCR (real-time RT-PCR), and various protocols therefor have been proposed worldwide. The genetic site used to detect the viruses differs from one protocol to another which necessitates the need for more academic evidence to determine optimal protocols.

Currently, COVID-19 testing is being implemented for those with fever or respiratory symptoms (cough, sore throat, etc.) within 14 days after visiting countries and regions where COVID-19 has been reported, as well as suspected patients who have been in close contact with confirmed patients.

Among patients infected with COVID-19, however, asymptomatic infection is being confirmed. Regardless of symptoms, antibody test methods reveal an immune response resulting from exposure to the virus, but are not useful for early diagnosis of COVID-19 because antibodies are formed 1-2 weeks after infection.

Therefore, there is a need for an effective diagnosis method that is superior to conventionally developed diagnosis methods in terms of sensitivity and specificity and can diagnose COVID-19 rapidly and accurately with great clinical efficacy.

### [Related Art Document]

### Korean Patent Number 10-2109196

Yu Sangheon et al., Analytical Chemistry, Vol. 92, no. 20, September 2020, pages 14139-14144 relates to development of a lateral flow strip for detection of SARS-CoV-2.

Annamalai Pazhanimuthu et al., medRxiv, April 2020, XP055892461 relates to a simple colorimetric molecular detection of novel coronavirus (COVID-19), a diagnostic tool for pandemic screening.

CN 112 195 275 relates to a primer group, kit and method for the LAMP combined detection of the influenza A virus, influenza B virus and novel coronavirus.

CN 111 270 021 relates to a primer pair for detecting novel coronavirus SARS-CoV-2, a probe, a composition, a kit and an application thereof.

### SUMMARY OF THE INVENTION

Leading to the present disclosure, intensive and thorough research, conducted into the prompt and accurate diagnosis of COVID-19, resulted in the development of primers and probes that can use RdRp (RNA-dependent RNA polymerase) gene and ORF3a gene of SARS-CoV-2 virus as target markers and accurately detect the genes with high sensitivity and specificity.

Therefore, an aspect of the present disclosure is to provide a composition for detection of SARS-CoV-2 virus gene, the composition including: the primer of SEQ ID NO: 1, the primer of SEQ ID NO: 2, and the probe of SEQ ID NO: 3; or the primer of SEQ ID NO: 4, the primer of SEQ ID NO: 5, and the probe of SEQ ID NO: 6.

Another aspect of the present disclosure is to provide a diagnostic kit for COVID 19, the kit including the composition of the present disclosure.

A further aspect of the present disclosure is to provide a method for providing information for diagnosis of COVID-19, the method comprising the steps of:
(1) isolating a nucleic acid from a specimen from a patient suspected of COVID-19;
(2) performing PCR amplification with the diagnostic kit of the present disclosure, with the isolated nucleic acid serving as a template; and
(3) identifying RdRp or ORF3a gene of SARS-CoV-2 in the amplicon obtained in step (2).

To achieve the goal, the present disclosure provides a composition for detection of SARS-CoV-2 virus gene, the composition including: the primer of SEQ ID NO: 1, the primer of SEQ ID NO: 2, and the probe of SEQ ID NO: 3; or the primer of SEQ ID NO: 4, the primer of SEQ ID NO: 5, and the probe of SEQ ID NO: 6.

Also, the present disclosure provides a diagnostic kit for COVID-19, the kit including the composition of the disclosure.

In addition, the present disclosure provides a method for providing information for diagnosis of COVID-19, the method including the steps of: (1) isolating a nucleic acid from a specimen from a patient suspected of COVID-19; (2) performing PCR amplification with the diagnostic kit of the present disclosure, with the isolated nucleic acid serving as a template; and (3) identifying RdRp or ORF3a gene of SARS-CoV-2 in the amplicon obtained in step (2).

In an embodiment of the present disclosure, the specimen in step (1) may be blood, a tissue, a cell, a serum, plasma, saliva, sputum, a urine, or feces.

In an embodiment of the present disclosure, the PCR amplification may be selected from the group consisting of conventional PCR (C-PCR), nested PCR (N-PCR), multiplex PCR, real-time PCR, real-time quantitative PCR, and reverse transcription PCR.

In an embodiment of the present disclosure, the RdRp gene of SARS-CoV-2 virus in step (3) may be identified using the primer of SEQ ID NO: 1, the primer of SEQ ID NO: 2, and the probe of SEQ ID NO: 3.

In an embodiment of the present disclosure, the ORF3a gene of SARS-CoV-2 virus in step (3) may be identified using the primer of SEQ ID NO: 4, the primer of SEQ ID NO: 5, and the probe of SEQ ID NO: 6.

Being able to specifically detect an RdRp or ORF3a gene of SARS-CoV-2 virus, with superiority to conventional detection methods in terms of detection specificity and sensitivity, the composition for detection of a SARS-CoV-2 viral gene and the diagnostic method for COVID-19 using same according to the present disclosure have the effect of promptly and accurately diagnosing COVID-19 through PCR in a simple manner.

### DETAILED DESCRIPTION OF THE EXEMPLARY EMBODIMENTS

The present disclosure provides a composition for detection of a SARS-CoV-2 viral gene, whereby prompt and accurate diagnosis can be made of infection with SARS-CoV-2 virus.

While studying the development of a technique for the diagnosis of COVID-19 caused by SARS-CoV-2 virus, the present inventors found the use of RdRp or ORF3a gene of SARS-CoV-2 virus as a target marker and identified that the RdRp or ORF3a gene is a sequence specific for SARS-CoV-2 virus and can serve as a primer and probe allowing for detection of SARS-CoV-2 viral infection with high sensitivity and accuracy.

In an embodiment of the present disclosure, the RdRp or ORF3a gene sequence was analyzed for conserved regions on the basis of various sequence information on SARS-CoV-2 viral genes and primers and probes composed of various base sequences capable of detecting the conserved regions were constructed. From the pool of the constructed different nucleotide sequences, selection was made of primers and probes which are far superior in sensitivity and specificity for the RdRp or ORF3a gene of SARS-CoV-2 virus to the others.

In detail, designed in the present disclosure were the primers and probes including nCoV_RdRp-126F primer (5'-GCTGGTTTTGCTAAATTC-3', SEQ ID NO: 1), nCoV_RdRp-226R primer (5'-GTGTGTCTCTTAACTACAA-3', SEQ ID NO: 2), and nCoV_RdRp-151P probe (5'-CTAATTGTTGTCGCTTCCAAGAA- 3', SEQ ID NO: 3) for detecting an RdRp gene of SARS-CoV-2 virus, and nCoV_3a-63F primer (5'-GGATGCTACTCCTTCAGA-3', SEQ ID NO: 4), nCoV_3a-225R primer (5'-CTTGGAGAGTGCTAGTTG-3', SEQ ID NO: 5), and nCoV_3a-100P probe (5'-ACGATACCGATACAAGCCTCACTC- 3', SEQ ID NO: 6) for detecting and ORF3a gene of SARS-CoV-2 virus.

Hence, the present disclosure provides a composition for detection of SARS-CoV-2 virus gene, the composition including: the primer of SEQ ID NO: 1, the primer of SEQ ID NO: 2, and the probe of SEQ ID NO: 3; or the primer of SEQ ID NO: 4, the primer of SEQ ID NO: 5, and the probe of SEQ ID NO: 6.

The primer of SEQ ID NO: 1, the primer of SEQ ID NO: 2, and the probe of SEQ ID NO: 3 can be used to detect an RdRp (RNA-dependent RNA polymerase) gene of SARS-CoV-2 virus while the primer of SEQ ID NO: 4, the primer of SEQ ID NO: 5, and the probe of SEQ ID NO: 6 can be used to detect an ORF3a gene of SARS-CoV-2 virus.

In the present disclosure, the RdRp (RNA-dependent RNA polymerase) gene sequence is represented by SEQ ID NO: 9 and the ORF3a gene sequence is represented by SEQ ID NO: 10.

According to an embodiment of the present disclosure, PCR was conducted using the primer of SEQ ID NO: 1, the primer of SEQ ID NO: 2, and the probe of SEQ ID NO: 3, all designed in the present disclosure, to amplify the 101 bp-long sequence of SEQ ID NO: 7 which accounts for an RdRp target region of SARS-CoV-2, thereby detecting the RdRp target region.

In addition, PCR was conducted using the primer of SEQ ID NO: 4, the primer of SEQ ID NO: 5, and the probe of SEQ ID NO: 6, all designed in the present disclosure, to amplify the 163 bp-long sequence of SEQ ID NO: 8 which accounts for an ORF3a target region of SARS-CoV-2, thereby detecting the ORF3a target region.

Moreover, in another embodiment of the present disclosure, a kit including the primers and probes of the present disclosure was found to exhibit higher sensitivity and specificity for detection of SARS-CoV-2 virus than conventional SARS-CoV-2 virus detection kits, as analytically assayed by PCR using the same kits.

Accordingly, the composition for detection of a SARS-CoV-2 viral gene according to the present disclosure contains the primers and probes of the present disclosure which allow the detection of an RdRp (RNA-dependent RNA polymerase) or ORF3a gene of SARS-CoV-2 virus at high sensitivity and accuracy and can be used to determine whether SARS-CoV-2 virus is present or absent in a sample to be analyzed and can also be used as a diagnostic composition for COVID-19.

Therefore, the present disclosure provides a composition for diagnosis of COVID-19, the composition including the composition for detection of a SARS-CoV-2 viral gene.

The detection or diagnostic composition of the present disclosure may contain ingredients useful for polymerase chain reaction (PCR) for example, a reaction buffer, dNTP, Mg²⁺ ions, and DNA polymerase in addition to the aforementioned primer pair and probe according to the present disclosure.

The reaction buffer may contain 1-10 mM Tris HCl, 10-40 mM KCl (pH 9.0) and dNTP may be at least one selected from dATP, dTTP, dGTP, and dCTP.

In addition, the composition may contain a stabilizer and/or a non-reactive dye for experiment's convenience, stabilization, and reactivity improvement.

Here, the non-reactive dye material aims to analyze or discriminate an amplicon, without affecting the polymerase chain reaction. The material meeting the condition may be a water-soluble dye as exemplified by rhodamine, TAMRA, lax, bromophenol blue, xylene cyanol, bromocresol red, and cresol red.

The composition may be provided in a liquid phase form and preferably in a dried form in order to enhance stability, the convenience of storage, and long-term storage. The drying may be carried out using a conventional drying method such as room temperature drying, heating drying, freeze drying, vacuum drying, etc. So long as it does not cause the composition to lose its ingredients, any drying method may be employed. The application of such drying methods may depend on kinds and amounts of the enzymes used.

The composition may be a mixed form of the ingredients in one reaction tube, followed by stabilization through a freezing or drying process, whereby a separate mixing process is not needed during the PCR to prevent the error caused by mixing, with the consequent improvement of stability, reactivity, and storage.

Except for the primer pair and probe, the ingredients employed in the composition, for example, the reaction buffer, dNTP, Mg²⁺ ions, and DNA polymerase may be commercially available.

In addition, the present disclosure provides a diagnostic kit for COVID-19, the kit including the composition of the present disclosure.

The diagnosis may be made by PCR (polymerase chain reaction) for determining whether a specimen to be analyzed contains SARS-CoV-2 viral gene, in detail, an RdRp or ORF3a gene of SARS-CoV-2 virus. Examples of the PCR include general PCR, reverse transcription polymerase chain reaction (RT-PCR), and real-time polymerase chain reaction (real-time PCR). In an embodiment of the present disclosure, real-time reverse transcription PCR is carried out.

The diagnostic kit of the present disclosure may further include a reverse transcriptase, a polymerase, a complementary DNA (cDNA) serving as a template DNA for PCR, and reaction reagents such as a buffer, etc., in addition the primer pair and probe of the present disclosure.

According to an embodiment of the present disclosure, the diagnostic kit of the present disclosure may be used for general PCR or real-time PCR. In this regard, PCR may be performed on the DNA genome of SARS-CoV-2 virus isolated from a sample to be analyzed, or a reverse transcription reaction may be separately conducted using a reverse transcriptase on the RNA genome of SARS-CoV-2 virus to synthesize complementary DNA (cDNA) which then serves as a template DNA for PCR.

PCR products can be isolated by a proper method such as agarose gel electrophoresis or capillary electrophoresis. When determined in terms of length by using the primer pair and probe of the present disclosure, the presence of the DNA of interest may lead to determining the infection of SARS-CoV-2 and preferably diagnosing COVID-19.

In the present disclosure, a probe capable of binding specifically to an RdRp or ORF3a gene of SARS-CoV-2 virus is used. The probe may be labeled with a fluorophore and a quencher at the ends thereof. In detail, the probe may have a fluorophore bound to the 5' end thereof and a quencher bound to the 3' end thereof. For example, the probe may be labeled with a fluorophore emitting certain wavelengths such as red, green, blue, etc. at the 5' end and a black hole quencher (BHQ) at the 3' end. When the fluorophore and the quencher are both bound to the probe, the quencher absorbs the light emitted by the quencher so that no fluorescent signals are detected. In contrast, the fluorophore is cleaved due to the exonuclease activity of DNA polymerase during the DNA synthesis and extension process, thereby allowing the detection of fluorescent signals. During PCR, such fluorescent signals can be measured in real time and on the basis of the increased fluorescent signals, a decision can be made of the presence or absence of the viral gene. This probe may be useful for real-time PCR.

Furthermore, the present disclosure provides a method for diagnosis of COVID-19 and a method for providing information for diagnosis of COVID-19.

In some particular embodiments, the present disclosure provides a method for providing information for diagnosis of COVID-19, the method including the steps of: (1) isolating a nucleic acid from a specimen from a patient suspected of COVID-19; (2) performing PCR amplification with the diagnostic kit of claim 4, with the isolated nucleic acid serving as a template; and (3) identifying an RdRp or ORF3a gene of SARS-CoV-2 in the amplicon obtained in step (2).

When the nucleic acid isolated from the specimen is RNA, the method may further include the step of synthesizing cDNA from the RNA and performing real-time PCR with the diagnostic kit of the present disclosure, with the synthesized cDNA serving as a template.

The specimen may be blood, a tissue, a cell, a serum, plasma, saliva, sputum, a urine, or feces obtained from mammals, but with no limitations thereto.

In addition, the RdRp (RNA-dependent RNA polymerase) or ORF3a gene of SARS-CoV-2 virus may be identified using any one method selected from capillary electrophoresis, DNA chip, gel electrophoresis, radiation assay, fluorescence assay, and phosphorescence assay.

As described above, the kit including the primer pair and probe designed in the present disclosure, that is, the primer of SEQ ID NO: 1, the primer of SEQ ID NO: 2, and the probe of SEQ ID NO: 3, which all bind specifically to an RdRp (RNA-dependent RNA polymerase) gene of SARS-CoV-2 virus; or the primer of SEQ ID NO: 4, the primer of SEQ ID NO: 5, and the probe of SEQ ID NO: 6, which all bind specifically to an ORF3a gene of SARS-CoV-2 virus can detect the SARS-CoV-2 viral gene and thus diagnose COVID-19 promptly with high sensitivity and accuracy.

Hence, a method for diagnosis of COVID-19 and a method for providing information for COVID-19 diagnosis, each using the composition for detection of a SARS-CoV-2 viral gene, designed in the present disclosure, can identify the SARS-CoV-2 viral gene promptly and accurately in an early stage and detect the same gene in real time, thus making it possible to monitor the change of symptoms according to drug administration. Furthermore, the methods can discriminate COVID-19 from other diseases at high specificity and as such, can be advantageously used for selecting more accurate therapies.

A better understanding of the present disclosure may be obtained through the following examples which are set forth to illustrate, but are not to be construed as limiting the present disclosure.

### <EXAMPLE 1>

### Design of Primers and Probes for Detecting Genes of SARS-CoV-2 Virus

On the basis of the genomes of SARS-CoV-2 viruses isolated from COVID-19 patients in Korea, RdRp (RNA-dependent RNA polymerase) and ORF3a genes of SARS-CoV-2 virus were selected as detection target genes. In brief, the genomes were sequenced to identify conserved regions. On the basis of the conserved regions, primers and probes were designed to specifically detect the genes.

**TABLE 1**

| Information on Sequences of Identified SARS-CoV-2 | | | |
|---|---|---|---|
| GenBank accession NO. | Strain | Isolation source | Geographic origin |
| MN908947 | Wuhan-Hu-1 | Human | China |
| MN985325 | 2019-nCoV/USA-WA1/2020 | Human | USA |
| MN975262 | 2019-nCoV_HKU-SZ- 005b_2020 | Human | China |
| MN988713 | 2019-nCoV/USA-IL1/2020 | Human | USA |
| MN938384 | 2019-nCoV_HKU-SZ- 002a_2020 | Human | China |

**TABLE 2**

| Primers and Probes Designed to Target RdRp and ORF3a Genes of SARS-CoV-2 | | | | | | |
|---|---|---|---|---|---|---|
| No | Gene | Name | Sequence | Lengt h | SE Q ID NO | Product Length(bp ) |
| 1 | RdRp | nCoV_RdRp -126F | GCTGGTTTTGCTAAATTC | 18 | 1 | 101 |
| | | nCoV_RdRp -226R | GTGTGTCTCTTAACTACAA | 19 | 2 | |
| | | nCoV_RdRp -151P | CTAATTGTTGTCGCTTCCAAGAA | 23 | 3 | |
| 2 | ORF3 a | nCoV_3a-63F | GGATGCTACTCCTTCAGA | 18 | 4 | 163 |
| | | nCoV_3a-225R | CTTGGAGAGTGCTAGTTG | 18 | 5 | |
| | | nCoV_3a-100P | ACGATACCGATACAAGCCTCACT C | 24 | 6 | |

**TABLE 3**

| PCR Amplicon Sequence for RdRp and ORF3a Target Genes of SARS-CoV-2 | | |
|---|---|---|
| No. | gene | Nucleotide Sequence of PCR Amplicon |
| 1 | RdRp | gctggttttgctaaattcctaaaaactaattgttgtcgcttccaagaaaaggacgaag atgacaatttaattgattcttactttgtagttaagagacacac (SEQ ID NO: 7) |
| 2 | ORF3a | ggatgctactccttcagattttgttcgcgctactgcaacgataccgatacaagcctca ctccctttcggatggcttattgttggcgttgcacttcttgctgtttttcagagcgctt ccaaaatcataaccctcaaaaagagatggcaactagcactctccaag (SEQ ID NO: 8) |

### <EXAMPLE 2>

### Assay for Sensitivity and Specificity of Primers and Probes for Detection of SARS-CoV-2

Experiments were carried out to figure out whether the primers and probes designed in Example 1 to detect the SARS-CoV-2 viral genes can diagnose SARS-CoV-2 virus infection. The experiments were proceeded as follows.

### <2-1> Specimen selection

o Selection criteria
   1) Persons at 16 years of age or older
   2) Mild patients with COVID-19 confirmed cases (0-4 scores on the NEWS scoring system)
   3) Inpatients who agreed in writing
o Exclusions
   (1) Persons who cannot take oral medicine
   (2) Pregnant or lactating women
   (3) Immunocompromised patients
   (4) Renal failure patients with CCR < 30 mL/min
   (5) Patients of liver disease with ALT or AST > 5 times ULN

### <2-2> Assay for detection specificity and sensitivity

Real-time RT-PCR tests were carried out for diagnosing COVID-19 in the patients who met the three conditions above. In this regard, real-time RT-PCR was performed using the primers and probes finally selected in the present disclosure due to the excellent sensitivity and specificity thereof (nCoV_RdRp-126F/nCoV_RdRp-226R/nCoV_RdRp-151P, and nCoV_3a-63F/nCoV_3a-225R/nCoV_3a-100P) and PowerChekTM 2019-nCoV Real-time PCR kit (Kogenebiotech), which was approved by the Korea Disease Control and Prevention Agency.

For a sensitivity assay, the real-time RT-PCR testing was performed in COVID-19 patients in 2020. In this regard, a total of 93 specimens including sputa, nasopharyngeal and oropharyngeal swabs, and saliva from 13 patients were subjected to real-time RT-PCR. For a specificity assay, real time RT-PCR was carried out with other viral specimens and strains collected by BEI as negative controls, and sputum, nasopharyngeal and oropharyngeal swab, and saliva specimens which were identified negative in COVID-19 testing. The real-time RT-PCR was performed in the conditions shown in Table 4, below.

**TABLE 4**

| Condition for Real-Time RT-PCR | | |
|---|---|---|
| Temp. | Time | Cycle |
| 50°C | 10min | 1 |
| 95°C | 30sec | 1 |
| 95°C | 5sec | 45 |
| 55°C | 30sec | |
| Fluorescence scan | | |
| 40°C | 30sec | 1 |

**TABLE 5**

| Detection Specificity Assay Result of Inventive Primers and Probes in Various Viral Specimens and Strains | | | |
|---|---|---|---|
| No. | virus | RdRp (Inventive primers and probe) | ORF3a (Inventive primers and probe) |
| 1 | Avian infectious bronchitis virus | Undetermined | Undetermined |
| 2 | Human Coronavirus NL63 | Undetermined | Undetermined |
| 3 | Canine coronavirus | Undetermined | Undetermined |
| 4 | MERS-Coronavirus | Undetermined | Undetermined |
| 5 | SARS-CoV | Undetermined | Undetermined |
| 6 | Human respiratory syncytial virus | Undetermined | Undetermined |
| 7 | Measles virus | Undetermined | Undetermined |
| 8 | Rhinovirus | Undetermined | Undetermined |
| 9 | Human astrovirus (HAstV) type 1 | Undetermined | Undetermined |
| 10 | Human astrovirus (HAstV) type 2 | Undetermined | Undetermined |
| 11 | *Klebsiella pneumoniae* Isolate 1 | Undetermined | Undetermined |
| 12 | *Klebsiella oxytoca* MIT 10-5244 | Undetermined | Undetermined |
| 13 | *Leptospira interrogans* | Undetermined | Undetermined |
| 14 | *Mycobacterium abscessus* | Undetermined | Undetermined |
| 15 | *Mycobacterium avium* | Undetermined | Undetermined |
| 16 | *Mycobacterium intracellulare* | Undetermined | Undetermined |
| 17 | *Staphylococcus aureus* MRSA | Undetermined | Undetermined |
| 18 | *Staphylococcus aureus* | Undetermined | Undetermined |
| 19 | *Streptococcus pneumoniae* | Undetermined | Undetermined |
| 20 | *Pseudomonas aeruginosa* | Undetermined | Undetermined |
| 21 | Influenza A virus | Undetermined | Undetermined |
| 22 | Influenza B virus | Undetermined | Undetermined |
| 23 | Influenza C virus | Undetermined | Undetermined |
| 24 | SARS-CoV2 | 31.52 | 32.19 |

**TABLE 6**

| Detection Specificity Assay Result of Inventive Primers and Probes in COVID-19 Negative Clinical Specimens | | | | | | |
|---|---|---|---|---|---|---|
| No. | Lab no. | Specimen | Kogene kit: cut off 35 | | RdRp (Inventi ve) | ORF3a (Invent ive) |
| | | | E gene | RdRP | | |
| 1 | 2020-179 | Nasopharynx | NA | NA | UD | UD |
| 2 | 2020-180 | Nasopharynx | NA | NA | UD | UD |
| 3 | 2020-181 | Nasopharynx | NA | 40.00 | UD | UD |
| 4 | 2020-182 | Nasopharynx | NA | NA | UD | UD |
| 5 | 2020-195 | Nasopharynx | NA | NA | UD | UD |
| 6 | 2020-195 | Sputum | NA | NA | UD | UD |
| 7 | 2020-201 | Sputum | NA | NA | UD | UD |
| 8 | 2020-344 | Nasopharynx | NA | NA | UD | UD |
| 9 | 2020-344 | Sputum | NA | NA | UD | UD |
| 10 | 2020-345 | sputum | NA | NA | UD | UD |
| 11 | 2020-348 | Sputum | NA | NA | UD | UD |
| 12 | 2020-349 | Nasopharynx | NA | NA | UD | UD |
| 13 | 2020-380 | Nasopharynx | NA | NA | UD | UD |
| 14 | 2020-380 | Sputum | NA | NA | UD | UD |
| 15 | 2020-381 | Nasopharynx | NA | NA | UD | UD |
| 16 | 2020-381 | Sputum | NA | NA | UD | UD |
| 17 | 2020-382 | Nasopharynx | NA | NA | UD | UD |
| 18 | 2020-382 | sputum | NA | NA | UD | UD |
| 19 | 2020-383 | Nasopharynx | NA | NA | UD | UD |
| 20 | 2020-383 | sputum | NA | NA | UD | UD |
| 21 | 2020-384 | Nasopharynx | NA | NA | UD | UD |
| 22 | 2020-384 | Sputum | NA | NA | UD | UD |
| 23 | 2020-385 | Nasopharynx | NA | NA | UD | UD |
| 24 | 2020-385 | Sputum | NA | NA | UD | UD |
| 25 | 2020-387 | Nasopharynx | NA | NA | UD | UD |
| 26 | 2020-387 | Sputum | NA | NA | UD | UD |
| 27 | 2020-388 | Nasopharynx | NA | NA | UD | UD |
| 28 | 2020-388 | Sputum | NA | NA | UD | UD |
| 29 | 2020-389 | Nasopharynx | NA | NA | UD | UD |
| 30 | 2020-390 | Nasopharynx | NA | NA | UD | UD |
| 31 | 2020-390 | Sputum | NA | NA | UD | UD |
| 32 | 2020-391 | Nasopharynx | NA | NA | UD | UD |
| 33 | 2020-391 | Sputum | NA | NA | UD | UD |
| 34 | 2020-392 | Nasopharynx | NA | NA | UD | 43.90 |
| 35 | 2020-393 | Nasopharynx | NA | NA | UD | 40.08 |
| 36 | 2020-394 | Nasopharynx | NA | NA | UD | UD |
| 37 | 2020-395 | Nasopharynx | NA | NA | UD | UD |
| 38 | 2020-395 | Sputum | NA | NA | UD | UD |
| 39 | 2020-745 | Nasopharynx | NA | NA | UD | 43.00 |
| 40 | 2020-746 | Nasopharynx | NA | NA | UD | UD |
| 41 | 2020-747 | Nasopharynx | NA | NA | UD | UD |
| 42 | 2020-748 | Nasopharynx | NA | NA | UD | UD |
| 43 | 2020-749 | Nasopharynx | NA | NA | UD | UD |
| 44 | 2020-750 | Nasopharynx | NA | NA | UD | UD |
| 45 | 2020-751 | Nasopharynx | NA | NA | UD | UD |
| 46 | 2020-917 | Saliva | NA | NA | UD | UD |
| 47 | 2020-918 | Saliva | NA | NA | UD | UD |
| 48 | 2020-919 | Saliva | NA | NA | UD | UD |
| 49 | 2020-920 | Saliva | NA | NA | UD | 40.71 |
| 50 | 2020-921 | Saliva | NA | NA | UD | UD |
| 51 | 2020-922 | Saliva | NA | NA | UD | UD |
| 52 | 2020N-01 | Nasopharynx | NA | NA | UD | UD |
| 53 | 2020N-02 | Nasopharynx | NA | NA | UD | UD |
| 54 | 2020N-03 | Nasopharynx | NA | NA | UD | UD |
| 55 | 2020N-04 | Nasopharynx | NA | NA | UD | UD |
| 56 | 2020N-05 | Nasopharynx | NA | NA | UD | UD |
| 57 | 2020N-06 | Nasopharynx | NA | NA | UD | UD |
| 58 | 2020N-07 | Nasopharynx | NA | NA | UD | UD |
| 59 | 2020N-08 | Nasopharynx | NA | NA | UD | UD |
| 60 | 2020N-10 | Nasopharynx | NA | NA | UD | UD |
| 61 | 2020-752 | Nasopharynx | NA | NA | UD | UD |
| 62 | 2020-753 | Nasopharynx | NA | NA | UD | UD |
| 63 | 2020-754 | Nasopharynx | NA | NA | UD | UD |
| 64 | 2020-755 | Nasopharynx | NA | NA | UD | UD |
| 65 | 2020-756 | Nasopharynx | NA | NA | UD | UD |
| 66 | 2020-757 | Nasopharynx | NA | NA | UD | UD |
| 67 | 2020-758 | Nasopharynx | NA | NA | UD | UD |
| 68 | 2020-759 | Nasopharynx | NA | NA | UD | UD |
| 69 | 2020-760 | Nasopharynx | NA | NA | UD | UD |
| 70 | 2020-761 | Nasopharynx | NA | NA | UD | UD |
| 71 | 2020-762 | Nasopharynx | NA | NA | UD | UD |
| 72 | 2020-763 | Nasopharynx | NA | NA | UD | UD |
| 73 | 2020-764 | Nasopharynx | NA | NA | UD | UD |
| 74 | 2020-765 | Nasopharynx | NA | NA | UD | UD |
| 75 | 2020-766 | Nasopharynx | NA | NA | UD | UD |
| 76 | 2020-767 | Nasopharynx | NA | NA | UD | UD |
| 77 | 2020-768 | Nasopharynx | NA | NA | UD | UD |
| 78 | 2020-895 | Saliva | NA | NA | UD | UD |
| 79 | 2020-896 | Saliva | NA | NA | UD | UD |
| 80 | 2020-897 | Saliva | NA | NA | UD | UD |
| 81 | 2020-898 | Saliva | NA | NA | UD | 40.68 |
| 82 | 2020-899 | Saliva | NA | NA | UD | UD |
| 83 | 2020-900 | Saliva | NA | NA | UD | UD |
| 84 | 2020-901 | Saliva | NA | NA | UD | 41.28 |
| 85 | 2020-902 | Saliva | NA | NA | UD | UD |
| 86 | 2020-903 | Saliva | NA | NA | UD | UD |
| 87 | 2020-905 | Saliva | NA | NA | UD | UD |
| 88 | 2020-907 | Saliva | NA | NA | UD | UD |

| | | | | | | |
|---|---|---|---|---|---|---|
| UD= Undetermined, NA= Not available | | | | | | |

As understood from the data of Table 5, detection was negative to viruses other than SARS-CoV-2 upon the assay using the primers and probes of the present disclosure. Only the SARS-CoV-2 virus was specifically detected with the primers and probes.

Moreover, as shown in Table 6, the COVID-19-negative clinical specimens were also determined to be negative as assayed using the primers and probes of the present disclosure.

On the other hand, PCR using the primers and probes of the present disclosure succeeded in detecting SARS-CoV-2 viral genes in all of the specimens from COVID-19 positive patients in 2020, as shown in Table 7, below.

**TABLE 7**

| Detection Sensitivity Assay Result of Inventive Primers and Probes for Various Specimens from COVID-19-Positive Patients | | | | | | | | |
|---|---|---|---|---|---|---|---|---|
| | Lab No. | Specimen | 2019-nCoV Kogene kit: cutoff 35 | | | | Inventive | |
| | | | IC | E | IC | RdRp | RdRp | ORF3a |
| 1 | 2020-107 (CoV52) | saliva | 20.16 | 29.28 | 20.74 | 30.47 | 26.51 | 26.45 |
| 2 | 2020-108 (CoV32) | sputum | 20.28 | UD | 20.29 | NA | UD | UD |
| 3 | 2020-108 (CoV32) | saliva | 20.53 | 33.13 | 20.17 | 33.51 | 29.25 | 28.90 |
| 4 | 2020-111 (CoV46) | naso | 19.73 | 20.44 | 20.51 | 21.70 | 18.49 | 18.05 |
| 5 | 2020-111 (CoV46) | sputum | 19.90 | 30.36 | 20.48 | 30.12 | 27.50 | 28.39 |
| 6 | 2020-111 (CoV46) | saliva | 19.93 | 36.46 | 20.83 | 38.99 | 34.52 | 34.49 |
| 7 | 2020-CoV01 | naso | 20.16 | 25.60 | 20.20 | 26.89 | 23.24 | 23.37 |
| 8 | 2020-CoV01 | oro | 20.27 | 34.30 | 20.22 | 34.96 | 30.46 | 30.48 |
| 9 | 2020-CoV01 | sputum | 20.45 | 32.84 | 20.28 | 33.25 | 29.21 | 29.20 |
| 10 | 2020-CoV02 | sputum | 20.68 | 36.96 | 20.41 | 37.33 | 31.18 | 32.37 |
| 11 | 2020-CoV03 | naso | 20.33 | 35.35 | 20.16 | 37.36 | 34.11 | 33.83 |
| 12 | 2020-CoV03 | sputum | 20.56 | UD | 20.13 | 39.77 | 32.70 | 34.72 |
| 13 | 2020-CoV04 | sputum | 20.31 | 35.70 | 20.18 | 36.38 | 31.63 | 31.74 |
| 14 | 2020-CoV05 | sputum | 20.47 | UD | 20.18 | 39.13 | 32.74 | 34.72 |
| 15 | 2020-CoV06 | sputum | 20.37 | UD | 20.30 | UD | 36.34 | 35.93 |
| 16 | 2020-CoV07 | oro | 20.28 | UD | 20.27 | UD | UD | UD |
| 17 | 2020-CoV08 | oro | 19.76 | UD | 19.47 | UD | UD | UD |
| 18 | 2020-CoV105 | sputum | 20.03 | UD | 19.95 | UD | UD | UD |
| 19 | 2020-CoV108 | naso | 20.46 | UD | 20.14 | UD | UD | UD |
| 20 | 2020-CoV111 | naso | 19.79 | 37.31 | 19.84 | 37.79 | 35.68 | 34.35 |
| 21 | 2020-CoV111 | oro | 20.33 | 32.99 | 19.06 | 32.50 | 30.00 | 27.29 |
| 22 | 2020-CoV111 | sputum | 20.05 | 38.38 | 19.72 | 36.90 | 29.92 | 32.90 |
| 23 | 2020-CoV120 | naso | 20.77 | 33.61 | 20.31 | 34.51 | 32.13 | 32.37 |
| 24 | 2020-CoV127 | sputum | 20.04 | 37.33 | 19.63 | 35.54 | 32.42 | 34.08 |
| 25 | 2020-CoV13 | naso | 20.14 | 28.03 | 20.75 | 29.02 | 25.18 | 25.21 |
| 26 | 2020-CoV13 | saliva | 20.17 | 33.01 | 20.81 | 34.19 | 29.56 | 29.66 |
| 27 | 2020-CoV14 | naso | 19.88 | 21.30 | 20.82 | 22.86 | 19.43 | 19.11 |
| 28 | 2020-CoV16 | naso | 20.45 | 30.84 | 20.33 | 31.60 | 27.52 | 27.75 |
| 29 | 2020-CoV16 | sputum | 20.35 | 33.61 | 20.13 | 34.88 | 31.36 | 31.05 |
| 30 | 2020-CoV17 | naso | 20.32 | 34.12 | 20.39 | 34.99 | 31.69 | 31.24 |
| 31 | 2020-CoV170 | naso | 19.80 | 38.24 | 19.59 | 39.47 | 36.90 | 37.22 |
| 32 | 2020-CoV170 | oro | 19.81 | 38.78 | 19.57 | UD | 36.19 | 35.64 |
| 33 | 2020-CoV170 | sputum | 20.25 | UD | 19.82 | 39.39 | 36.57 | UD |
| 34 | 2020-CoV18 | naso | 19.92 | 26.35 | 19.53 | 27.03 | 23.62 | 23.79 |
| 35 | 2020-CoV18 | sputum | 19.75 | 24.38 | 19.61 | 24.86 | 21.48 | 21.42 |
| 36 | 2020-COV193 | naso | 19.81 | 37.81 | 19.60 | 39.50 | 36.20 | 36.70 |
| 37 | 2020-COV193 | sputum | 20.02 | 31.34 | 19.78 | 30.41 | 26.21 | 26.37 |
| 38 | 2020-COV194 | naso | 20.31 | UD | 19.06 | 38.23 | 35.06 | 35.28 |
| 39 | 2020-COV194 | sputum | 21.05 | 39.55 | 21.00 | 36.52 | 31.46 | UD |
| 40 | 2020-COV224 | sputum | 20.27 | UD | 20.09 | 39.24 | 35.05 | 34.35 |
| 41 | 2020-CoV27 | sputum | 20.12 | 34.08 | 20.67 | 34.65 | 31.58 | 31.17 |
| 42 | 2020-COV287 | naso | 20.20 | 34.65 | 20.29 | 35.35 | 32.43 | 33.29 |
| 43 | 2020-COV287 | sputum | 20.73 | 36.96 | 20.53 | 36.93 | 33.87 | 36.10 |
| 44 | 2020-COV288 | sputum | 20.38 | 27.20 | 20.32 | 26.00 | 23.54 | 25.98 |
| 45 | 2020-COV300 | naso | 20.34 | UD | 20.23 | UD | UD | 37.35 |
| 46 | 2020-COV300 | sputum | 20.29 | 36.53 | 20.36 | 34.32 | 32.59 | 35.73 |
| 47 | 2020-COV329 | sputum | 20.31 | 35.88 | 20.19 | 37.70 | 35.31 | 34.04 |
| 48 | 2020-CoV33 | sputum | 20.60 | 39.76 | 20.47 | 39.25 | 36.97 | 43.81 |
| 49 | 2020-CoV33 | sputum | 20.36 | 24.65 | 20.19 | 25.21 | 21.08 | 21.39 |
| 50 | 2020-CoV34 | saliva | 20.64 | 35.39 | 20.33 | 37.40 | 33.05 | 33.13 |
| 51 | 2020-CoV36 | naso | 20.28 | 28.47 | 20.37 | 29.77 | 26.21 | 27.06 |
| 52 | 2020-COV376 | sputum | 20.34 | 38.43 | 20.21 | 39.12 | 39.24 | 36.69 |
| 53 | 2020-CoV38 | naso | 20.32 | 31. 07 | 20.24 | 32.31 | 28.75 | 27.59 |
| 54 | 2020-CoV39 | sputum | 20.17 | 35.35 | 20.06 | 37.02 | 33.27 | 33.22 |
| 55 | 2020-CoV41 | naso | 20.30 | 37.34 | 20.31 | 36.57 | 32.82 | 33.61 |
| 56 | 2020-CoV47 | oro | 20.23 | 37.53 | 20.70 | 39.10 | 39.15 | 36.32 |
| 57 | 2020-CoV48 | naso | 20.42 | 37.15 | 20.59 | 37.51 | 34.63 | 34.39 |
| 58 | 2020-CoV48 | oro | 20.37 | 28.50 | 20.69 | 29.26 | 26.50 | 26.26 |
| 59 | 2020-CoV49 | naso | 20.04 | 38.33 | 20.46 | 39.36 | 35.85 | 35.54 |
| 60 | 2020-CoV51 | oro | 20.10 | 38.07 | 20.78 | 37.18 | 34.84 | 35.24 |
| 61 | 2020-CoV54 | saliva | 20.18 | 28.78 | 20.92 | 30.14 | 25.75 | 26.17 |
| 62 | 2020-CoV55 | naso | 20.16 | 27.13 | 21.04 | 28.69 | 24.92 | 24.77 |
| 63 | 2020-CoV55 | oro | 20.11 | 28.07 | 21.06 | 29.75 | 25.25 | 25.06 |
| 64 | 2020-CoV55 | saliva | 20.40 | 34.22 | 21.08 | 36.01 | 31.14 | 30.25 |
| 65 | 2020-CoV56 | sputum | 20.34 | 29.90 | 20.75 | 26.19 | 24.23 | 26.10 |
| 66 | 2020-CoV56 | saliva | 20.21 | 34.26 | 20.46 | 35.55 | 31.45 | 31.01 |
| 67 | 2020-CoV57 | naso | 20.00 | 29.35 | 20.51 | 30.53 | 25.39 | 25.56 |
| 68 | 2020-CoV57 | oro | 20.18 | 36.18 | 20.68 | 37.99 | 34.69 | 34.14 |
| 69 | 2020-CoV57 | sputum | 20.36 | 28.97 | 20.48 | 29.40 | 26.28 | 26.09 |
| 70 | 2020-CoV57 | saliva | 20.18 | 27.47 | 20.59 | 28.86 | 24.41 | 23.71 |
| 71 | 2020-CoV58 | sputum | 20.14 | 35.14 | 20.76 | 35.28 | 31.92 | 31.39 |
| 72 | 2020-CoV58 | saliva | 20.27 | 36.29 | 21.01 | UD | 35.00 | 33.94 |
| 73 | 2020-CoV61 | sputum | 20.07 | 34.88 | 20.77 | 33.91 | 31.14 | 31.34 |
| 74 | 2020-CoV64 | sputum | 20.01 | 29.13 | 19.45 | 28.78 | 24.53 | 24.47 |
| 75 | 2020-CoV88 | naso | 19.86 | 31.66 | 19.53 | 31.58 | 28.66 | 28.86 |
| 76 | 2020-CoV88 | sputum | 20.34 | 35.24 | 20.02 | 35.28 | 31.60 | 30.86 |
| 77 | 2020-CoV91 | naso | 20.04 | 18.73 | 20.10 | 19.83 | 16.75 | 17.21 |
| 78 | 2020-CoV92 | naso | 19.96 | 33.67 | 19.82 | 34.46 | 31.39 | 31.15 |
| 79 | 2020-CoV92 | sputum | 20.01 | 31.53 | 19.77 | 29.78 | 24.80 | 25.66 |
| 80 | 2020-CoV93 | naso | 19.79 | 33.40 | 19.70 | 34.68 | 31.48 | 30.56 |
| 81 | 2020-CoV93 | sputum | 20.41 | 34.38 | 20.02 | 34.79 | 31.27 | 30.51 |
| 82 | 2020-CoV93 | saliva | 20.01 | UD | 19.63 | UD | UD | 43.02 |
| 83 | 2020-CoV94 | sputum | 19.97 | 35.30 | 19.67 | 35.46 | 30.92 | 30.31 |
| 84 | 2020-CoV95 | naso | 19.77 | UD | 19.48 | UD | 36.12 | UD |
| 85 | 2020-CoV95 | sputum | 20.03 | UD | 19.89 | 39.31 | 36.14 | 35.09 |
| 86 | 2020-CoV96 | naso | 19.63 | 36.45 | 19.62 | 37.32 | 35.14 | 35.17 |
| 87 | 2020-CoV96 | oro | 19.88 | 38.97 | 19.67 | UD | 36.61 | UD |
| 88 | 2020-CoV97 | naso | 20.20 | 26.90 | 20.28 | 27.81 | 25.02 | 25.28 |
| 89 | 2020-CoV97 | sputum | 20.36 | 23.95 | 20.14 | 24.33 | 21.10 | 21.66 |
| 90 | 2020-CoV97 | naso | 19.88 | 25.20 | 19.67 | 24.48 | 21.13 | 22.37 |
| 91 | 2020-CoV97 | oro | 19.80 | 23.64 | 19.73 | 24.52 | 20.99 | 20.95 |
| 92 | 2020-CoV97 | sputum | 19.85 | 23.25 | 19.63 | 22.20 | 18.52 | 20.19 |
| 93 | 2020-CoV98 | sputum | 20.06 | 24.73 | 19.98 | 24.97 | 21.24 | 20.90 |

| | | | | | | | | |
|---|---|---|---|---|---|---|---|---|
| UD= Undetermined | | | | | | | | |

From the data, it is understood that the primers and probes designed to target RdRp and ORF3a genes of SARS-CoV-2 virus according to the present disclosure can detect SARS-CoV-2 virus at high specificity.

Furthermore, PCR using the primers and probes of the present disclosure was assayed for sensitivity in specimens from COVID-19 patients in 2020.

**TABLE 8**

| Detection Sensitivity Assay Result of Inventive Primers and Probes Various Specimens from COVID-19-Positive Patients | | | | | | | | | | | | |
|---|---|---|---|---|---|---|---|---|---|---|---|---|
| | cutoff ≤ 35 | cutoff ≤ 35 | cutoff ≤ 40 | cutoff ≤ 40 | cutoff ≤ 35 | cutoff ≤ 35 | | | | | | |
| Detection kit | Kogene E | Kogene RdRp | Inventive kit lab | Inventive kit | Inventive kit lab | Inventive kit | | | | | | |

| | | | | | RdRp | | lab ORF3a | | RdRp | | lab ORF3a | |
|---|---|---|---|---|---|---|---|---|---|---|---|---|
| | + centre 1 | centre 1 | + cont rol | contr ol | + contr ol | contr ol | + contr ol | contr ol | + contr ol | contr ol | + contr ol | contr ol |
| Positive | 48 | 0 | 46 | 0 | 86 | 0 | 82 | 0 | 68 | 0 | 68 | 0 |
| Negative | 45 | 88 | 47 | 88 | 7 | 88 | 11 | 88 | 25 | 88 | 25 | 88 |
| Sensitivity (%) | 51.6 | | 49.5 | | 92.5 | | 88.2 | | 73.1 | | 73.1 | |
| Confidence interval(%) | 41.1-62.0 | | 39.0-56.0 | | 84.6-96.7 | | 79.4-93.7 | | 62.8-81.5 | | 62.8-81.5 | |
| Specificity (%) | 100 | | 100 | | 100 | | 100 | | 100 | | 100 | |
| Confidence interval (%) | 94.8-100 | | 94.8-100 | | 94.8-100 | | 94.8-100 | | 94.8-100 | | 94.8-100 | |

As is understood from the data of Table 8, the detection kits using the primers and probes designed in the present disclosure exhibited far higher detection sensitivity than conventional detection kits used as controls.

## Claims

1. A composition for detection of SARS-CoV-2 viral gene, the composition comprising: a primer of SEQ ID NO: 1, a primer of SEQ ID NO: 2, and a probe of SEQ ID NO: 3; or a primer of SEQ ID NO: 4, a primer of SEQ ID NO: 5, and a probe of SEQ ID NO: 6.

2. A diagnostic kit for corona virus disease-19 (COVID-19), the kit comprising the composition of claim 1.

3. A method for providing information for diagnosis of COVID-19, the method comprising the steps of:
(1) isolating a nucleic acid from a specimen from a patient suspected of COVID-19;
(2) performing PCR amplification with the diagnostic kit of claim 2, with the isolated nucleic acid serving as a template; and
(3) identifying RdRp or ORF3a gene of SARS-CoV-2 in the amplicon obtained in step (2).

4. The method of claim 3, wherein the specimen in step (1) is blood, a tissue, a cell, a serum, plasma, saliva, sputum, a urine, or feces.

5. The method of claim 3, wherein the PCR amplification is selected from the group consisting of conventional PCR (C-PCR), nested PCR (N-PCR), multiplex PCR, real-time PCR, real-time quantitative PCR, and revers transcription PCR.

6. The method of claim 3, wherein the RdRp gene of SARS-CoV-2 virus in step (3) is identified using the primer of SEQ ID NO: 1, the primer of SEQ ID NO: 2, and the probe of SEQ ID NO: 3.

7. The method of claim 3 wherein the ORF3a gene of SARS-CoV-2 virus in step (3) is identified using the primer of SEQ ID NO: 4, the primer of SEQ ID NO: 5, and the probe of SEQ ID NO: 6.

## Patentansprüche

1. Eine Zusammensetzung zum Nachweis des SARS-CoV-2-Viralgens, wobei die Zusammensetzung Folgendes beinhaltet: einen Primer von SEQ ID NO: 1, einen Primer von SEQ ID NO: 2 und eine Sonde von SEQ ID NO: 3; oder einen Primer von SEQ ID NO: 4, einen Primer von SEQ ID NO: 5 und eine Sonde von SEQ ID NO: 6.

2. Ein Diagnose-Kit für die Coronavirus-Krankheit-19 (COVID-19), wobei das Kit die Zusammensetzung gemäß Anspruch 1 beinhaltet.

3. Ein Verfahren zum Bereitstellen von Informationen zur Diagnose von COVID-19, wobei das Verfahren die folgenden Schritte beinhaltet:
(1) Isolieren einer Nukleinsäure aus einer Probe von einem Patienten, bei dem der Verdacht besteht, dass er an COVID-19 leidet;
(2) Durchführen einer PCR-Amplifikation mit dem Diagnose-Kit gemäß Anspruch 2, wobei die isolierte Nukleinsäure als eine Matrize dient; und
(3) Identifizieren des RdRp- oder ORF3a-Gens von SARS-CoV-2 in dem Amplikon, das in Schritt (2) erhalten wird.

4. Verfahren gemäß Anspruch 3, wobei es sich bei der Probe in Schritt (1) um Blut, ein Gewebe, eine Zelle, ein Serum, Plasma, Speichel, Sputum, Urin oder Fäzes handelt.

5. Verfahren gemäß Anspruch 3, wobei die PCR-Amplifikation aus der Gruppe ausgewählt ist, die aus Folgendem besteht: konventioneller PCR (C-PCR), verschachtelter PCR (N-PCR), Multiplex-PCR, Echtzeit-PCR, quantitativer Echtzeit-PCR und Reverser-Transkriptase-PCR.

6. Verfahren gemäß Anspruch 3, wobei das RdRp-Gen des SARS-CoV-2-Virus in Schritt (3) unter Verwendung des Primers von SEQ ID NO: 1, des Primers von SEQ ID NO: 2 und der Sonde von SEQ ID NO: 3 identifiziert wird.

7. Verfahren gemäß Anspruch 3, wobei das ORF3a-Gen des SARS-CoV-2-Virus in Schritt (3) unter Verwendung des Primers von SEQ ID NO: 4, des Primers von SEQ ID NO: 5 und der Sonde von SEQ ID NO: 6 identifiziert wird.

## Revendications

1. Une composition pour la détection d'un gène viral du SARS-CoV-2, la composition comprenant : une amorce de SEQ ID NO : 1, une amorce de SEQ ID NO : 2, et une sonde de SEQ ID NO : 3 ; ou une amorce de SEQ ID NO : 4, une amorce de SEQ ID NO : 5, et une sonde de SEQ ID NO : 6.

2. Un kit de diagnostic pour la maladie à coronavirus 2019 (COVID-19), le kit comprenant la composition de la revendication 1.

3. Un procédé pour la fourniture d'informations pour le diagnostic de la COVID-19, le procédé comprenant les étapes suivantes :
(1) isolement d'un acide nucléique issu d'un prélèvement provenant d'un patient suspect de COVID-19 ;
(2) réalisation d'une amplification par PCR avec le kit de diagnostic de la revendication 2, l'acide nucléique isolé servant de matrice ; et
(3) identification du gène RdRp ou ORF3a du SARS-CoV-2 dans l'amplicon obtenu à l'étape (2).

4. Le procédé de la revendication 3, dans lequel le prélèvement à l'étape (1) est du sang, un tissu, une cellule, un sérum, du plasma, de la salive, des expectorations, une urine, ou des matières fécales.

5. Le procédé de la revendication 3, dans lequel l'amplification par PCR est sélectionnée dans le groupe constitué de la PCR classique (C-PCR), la PCR nichée (N-PCR), la PCR multiplex, la PCR en temps réel, la PCR quantitative en temps réel, et la PCR après transcription inverse.

6. Le procédé de la revendication 3, dans lequel le gène RdRp du virus SARS-CoV-2 à l'étape (3) est identifié à l'aide de l'amorce de SEQ ID NO : 1, l'amorce de SEQ ID NO : 2, et la sonde de SEQ ID NO : 3.

7. Le procédé de la revendication 3 dans lequel le gène ORF3a du virus SARS-CoV-2 à l'étape (3) est identifié à l'aide de l'amorce de SEQ ID NO : 4, l'amorce de SEQ ID NO : 5, et la sonde de SEQ ID NO : 6.
